# EUROPÄISCHE PATENTSCHRIFT

(11) **EP 0 727 188 B1**
(45) Veröffentlichungstag und Bekanntmachung des Hinweises auf die Patenterteilung: **11.11.1998**
(21) Anmeldenummer: 95810106.5
(22) Anmeldetag: 17.02.1995
(51) Int. Cl.: A61B 17/60

(54) **Verbindungssystem für Pedikelschrauben**
Connection system for pedicle screws
Système de connection pour vis pédiculaires

(43) Veröffentlichungstag der Anmeldung: 21.08.1996
(73) Patentinhaber: Sulzer Orthopädie AG, 6340 Baar (CH)
(72) Erfinder: Baumgartner, Walter, CH-9500 Wil (CH); Freudiger, Stefan, CH-3047 Bremgarten (CH); Dubois, Gilles, c/o Nouvelle Clinique de l'Union, F-31240 St. Jean (FR)
(74) Vertreter: Hammer, Bruno Dr.

(56) Entgegenhaltungen:
- WO-A-93/01772

## Beschreibung

Die Erfindung handelt gemäss Oberbegriff vom Patentanspruch 1 von einem Verbindungssystem für Pedikelschrauben, die in verschiedenen Wirbeln verankerbar sind, mit einem durchbrochenen Verbindungsträger, mit Pedikelschrauben, die einen Kopf mit kugelförmiger Abstützfläche aufweisen, mit einem durchbrochenen Gegenkörper, der am Verbindungsträger aufliegt und auf der Gegenseite eine kalottenförmige Abstützfläche mit Durchbruch aufweist, und mit einem Schraubelement, welches eine Kugelabstützfläche besitzt und über ein Gewinde in der Richtung der Achse der Pedikelschraube letztere mit dem Verbindungsträger und dem Gegenkörper verspannt.

Pedikelschrauben, die in verschiedenen Rückenwirbeln eingeschraubt sind, dienen in der Regel als Basis für die Befestigung von Stützelementen und Trägern zwischen den Pedikelschrauben um Lagekorrekturen zwischen Rückenwirbeln vorzunehmen oder um eine Entlastung von Bandscheiben und eine Versteifung zwischen benachbarten Wirbeln vorzunehmen.

Ein Verbindungssystem für Pedikelschrauben ist in der Patentschrift US 5,129,899 gezeigt. Sie zeigt einen flachen, mit einem über seine Länge schlitzförmig ausgebildeten Durchbruch versehenen Träger, der sich über zwei und mehr Wirbel erstreckt. Die beiden durch den Schlitz getrennten Trägerarme besitzen auf ihren äusseren Seitenflächen eine Zahnung, an der Unterlagscheiben, die herabgezogene Seitenbacken mit einer Gegenzahnung aufweisen, in variablen Abständen in der Längsrichtung positioniert werden können. Die übergreifenden Seitenbacken haben dabei den Vorteil, dass sie ein Auseinanderspreizen der beiden Trägerarme verhindern, da Trägerarme und Unterlagscheiben zwischen kugelförmigen Abstützflächen an Pedikelschraube und an Befestigungsmutter verspannt sind. Die Anordnung hat den Nachteil, dass die Pedikelschrauben mit ihren Achsen in einer gemeinsamen Ebene liegen müssen, damit beim Befestigen des verbindenden Trägers zu einer selbsttragenden Konstruktion keine bleibenden Torsionsmomente im Träger erzeugt werden, die ihrerseits auf die Verankerung der Pedikelschrauben im Knochen wirken.

Aufgabe der Erfindung ist es eine einfache Verbindung zwischen windschief zueinanderstehenden Pedikelschrauben zu schaffen. Diese Aufgabe wird mit den Kennzeichen des Anspruchs 1 gelöst. Dadurch, dass das Schraubelement eine Scraube ist, die mit ihrem Schaft im Kopf der Pedikelschraube verankerbar ist,dass der Gegenkörper auf der der kalottenförmigen Abstützfläche gegenüberliegenden Seite auf einer ebenen mit dem Verbindungsträger gemeinsamen Stützfläche beliebig verschiebbar ist, um die Pedikelschraube mit ihrer Achse in einem von der Senkrechten auf die ebene Stützfläche abweichenden Winkel über den Gegenkörper am Verbindungsträger zu befestigen, wobei in der Schrägstellung aus der Senkrechten zur ebenen Stützfläche Gegenkörper und Verbindungsträger selbsthemmend verbindbar sind, entsteht eine spannungsfreie Verbindung. Diese Anordnung hat den Vorteil, dass selbstschneidende Pedikelschrauben verwendbar sind, die ihre Achsrichtung während dem Einschrauben entsprechend den Härteunterschieden von Knochengewebe noch verändern, um zu einer optimalen Verankerung zu kommen. Diese Richtungsunterschiede sowie die Unterschiede in der Ausrichtung der Knochenpartien für die Verankerung der Pedikelschrauben werden durch das Verbindungssystem kompensiert. Weiter vorteilhafte Ausbildungen der Erfindung sind in den abhängigen Ansprüchen 2 bis 12 aufgezeigt.

Um die Distanzunterschiede zwischen den Köpfen von zwei und mehr in verschiedenen Wirbeln verankerten Pedikelschrauben zu überbrücken sind Verbindungsträger mit Langlöchern vorgesehen, die in der Richtung ihrer Längsachse zylindrisch verlaufende Abstützflächen für die Kugelabstützflächen der Schraubelemente aufweisen, wobei sich Kugel- und Zylinderradius entsprechen. Entsprechend einem Baukastensystem sind Verbindungsträger vorgesehen deren Langlöcher unterschiedliche mittlere Abstände aufweisen, um so einen ganzen Bereich für Abstände der Köpfe von Pedikelschrauben abzudecken. Eine weitere Variation entsteht dadurch, dass an einem Verbindungsträger die im Bereich der Langlöcher ebenen Abstützflächen für die Gegenkörper zueinander parallel, parallel und versetzt oder nicht parallel sein können Eine weitere Variation besteht darin, dass die Längsachsen der Langlöcher in der senkrechten Projektion auf die Ebene einer Abstützfläche zueinander versetzt sein können. Um für diese letzten beiden Variationen keine zusätzlichen Verbindungsträger an Lager halten zu müssen, sind die Bereiche der Verbindungsträger zwischen zwei Langlöchern so geschwächt, dass sie mit Werkzeugen im Operationssaal in eine entsprechende Übergangsform zwischen den Abstützflächen verbiegbar sind. Dadurch dass die Achse jeder Pedikelschraube aus der Senkrechten zur ebenen Abstützfläche in jeder Richtung um einen Winkel schräg stehen kann, welcher eine durch die Formgebung unterstützte Selbsthemmung der Verbindungen zwischen dem Kopf der Pedikelschraube und dem Verbindungsträger gewährleistet, lässt sich die Genauigkeit für die Verformung der Verbindungsträger und die Anzahl der verschiedenen Verbindungsträger in vernünftigen Grenzen halten. Das heisst eine grobe Stufung ist möglich. Die Selbsthemmung wird allgemein dadurch vergrössert, dass mindestens eine der miteinander gepaarten Abstützflächen aufgerauht ist oder eine Mikrostruktur mit vorstehenden Spitzen aufweist, um über plastische Deformation einen Formschluss zu erreichen.

Eine weitere Massnahme besteht darin, einen möglichst grossen Teil des später unter Belastung zwischen Verbindungsträger und Pedikelschraube auftretenden Biegemomentes über die kalottenförmige Abstützfläche des Gegenkörpers auf die kugelförmige Abstützfläche am Kopf der Pedikelschraube zu übertragen. Dies geschieht dadurch, dass der Gegenkörper als Ring ausgeführt ist, dessen Kalotte gegenüber der Kugelfläche der Pedikelschraube Untermass d.h. einen kleineren Radius aufweist. Die Kugel, die zunächst am äusseren Rand der Kalotte aufsitzt, wird durch das Zusammenziehen in die Kalotte hineingedrückt, welche sich unter der Keilwirkung der Kugel elastisch nach aussen ausdehnt bis praktisch die ganze Kalottenfläche an der Kugel anliegt. Es entsteht so ein Maximum an ineinandergreifenden Mikrostrukturen der beiden Oberflächen und eine entsprechend drehsichere Verbindung in jeder Richtung.

In einer weiteren Ausgestaltung der Erfindung wird der zulässige Schwenkbereich der Pedikelschrauben aus der Senkrechten zur ebenen Stützfläche am Verbindungsträger erheblich durch eine sich im Formschluss gegenseitig durchdringende Mikrostruktur von Verbindungsträger und Gegenkörper vergrössert. Die Stützflächen können durch die Wiederholung ihrer Struktur in einem feinen Raster ineinander eintauchen und lassen sich mindestens in zwei Richtungen in kleinen Schritten verschieben, wobei zum Lösen der Verbindung im Rahmen der Mikrostruktur eine Herausheben und nach dem Verschieben ein Wiedereintauchen notwendig ist, um in eingerasteter Stellung Schubkräfte zu übertragen. Typische Raster können auf der einen Stützfläche aus regelmässig angeordneten Pyramiden mit einer Grundfläche aus Quadraten ober Rhomben sein, weil deren Herstellung z.B. durch Fräsen oder Schleifen besonders einfach ist, wobei auf gleiche Weise die Form an einem Presswerkzeug erzeugt wird, welches durch die Formumkehr beim Pressen die Gegenfläche erzeugt. Statt Pyramiden können auch Kegel oder andere Körper im gleichen Raster angeordnet sein. Der Vorteil der Anschrägung der sich durchdringenden Mikrostrukturen besteht darin, dass sie sich unter Eigengewicht von Verbindungsträger oder Gegenkörper in der gemeinsamen ebenen Abstützfläche noch zueinander verschieben lassen und trotzdem unter einer schräg zur Abstützfläche stehenden Anpresskraft eine starre Verbindung bilden.

In einer weiteren Anordnung sind an den Verbindungsträgern Langlöcher ohne Ansenkungen vorgesehen und ist dafür auf der der Pedikelschraube abgekehrten Seite ein weiterer Gegenkörper zum Schraubelement angebracht, dessen Kugelabstützfläche mit der kalottenförmigen Abstützfläche des Gegenkörpers korrespondiert. Die tragenden Stützflächen weisen ebenfalls, wie oben beschrieben, eine sich durchdringende Mikrostruktur auf. Die oben beschriebenen Variationen der Verbindungsträger sind ebenfalls möglich.

Im folgenden sind Ausführungsbeispiele für die Erfindung aufgeführt. Es zeigen:
- Fig. 1: schematisch die Verbindung von zwei zueinander windschief stehenden Pedikelschrauben;
- Fig. 2a: schematisch einen vergrösserten Schnitt durch den Kopf einer Pedikelschraube und dessen Verbindung mit einem Verbindungsträger gemäss Fig. 1;
- Fig. 2b: schematisch einen vergrösserten Schnitt durch eine Anordnung mit zwei Gegenkörpern, die von beiden Seiten am Langloch des Verbindungsträgers anliegen;
- Fig. 3: schematisch einen vergrösserten Längsschnitt durch einen Verbindungsträger gemäss Fig. 1;
- Fig. 4: schematisch eine Draufsicht auf einen Verbindungsträger gemäss Figur 3;
- Fig. 5: schematisch und stark vergrössert die Aufweitung von einem Gegenkörper mit Untermass durch die kugelförmige Abstützfläche einer Pedikelschraube;
- Fig. 6: schematisch einen Verbindungsträger mit drei Langlöchern deren zugehörige Stützflächen auf unterschiedlichem Niveau angeordnet sind; und
- Fig. 7: schematisch vergrösserte Mikrostrukturen für die Stützflächen zwischen Verbindungsträger und Gegenkörper, wobei die Gegenformen, wie sie aus einem Presswerkzeug ausfallen würden, gezeigt sind.

Die Figuren zeigen ein Verbindungssystem für Pedikelschrauben, die in verschiedenen Wirbeln verankerbar sind. Die kugelförmigen Schraubenköpfe liegen in Kugelschalen von durchbrochenen Gegenkörpern auf, die ihrerseits mit ebenen Abstützflächen an einem Verbindungsträger im Bereich von Langlöchern aufliegen. In der Achsrichtung der Pedikelschrauben ist jeweils ein Schraubelement vorgesehen, das mit einer Kugelabstützfläche im Bereich des Langloches am Verbindungsträger schwenkbar gelagert ist. Die Pedikelschrauben, die im allgemeinen windschief zueinander stehen, wenn auf die Knochenbeschaffenheit Rücksicht genommen wird, lassen sich über die Schraubelemente mit den Verbindungsträgern starr verbinden, weil auf bei zu den Abstützflächen schräg stehenden Pedikelschrauben, die Abstützflächen selbsthemmend gegeneinander verspannbar sind.

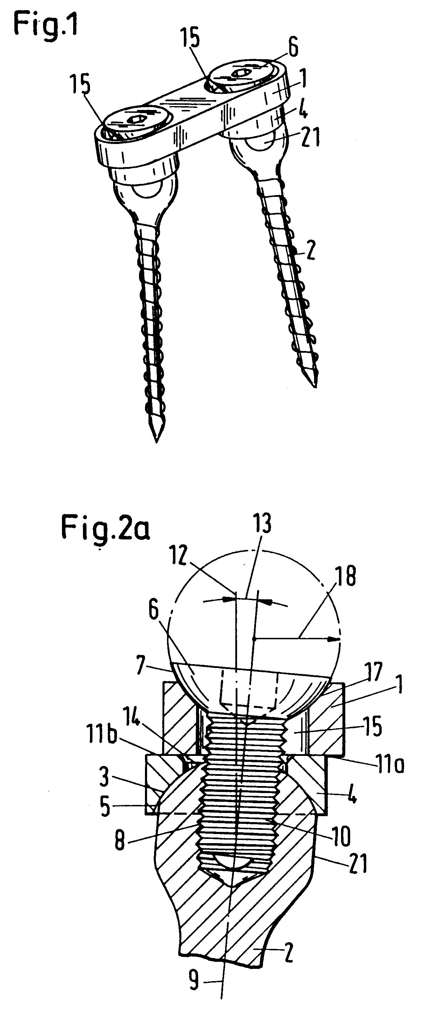

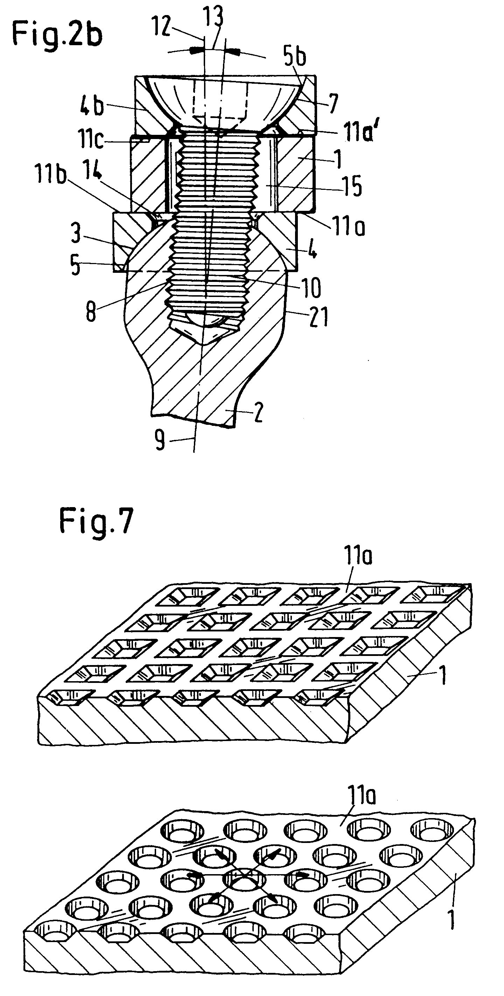

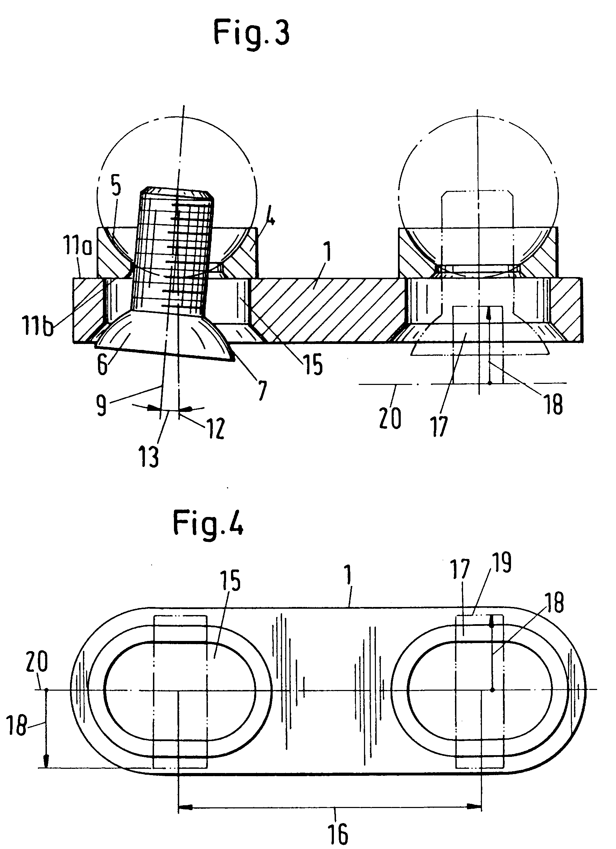

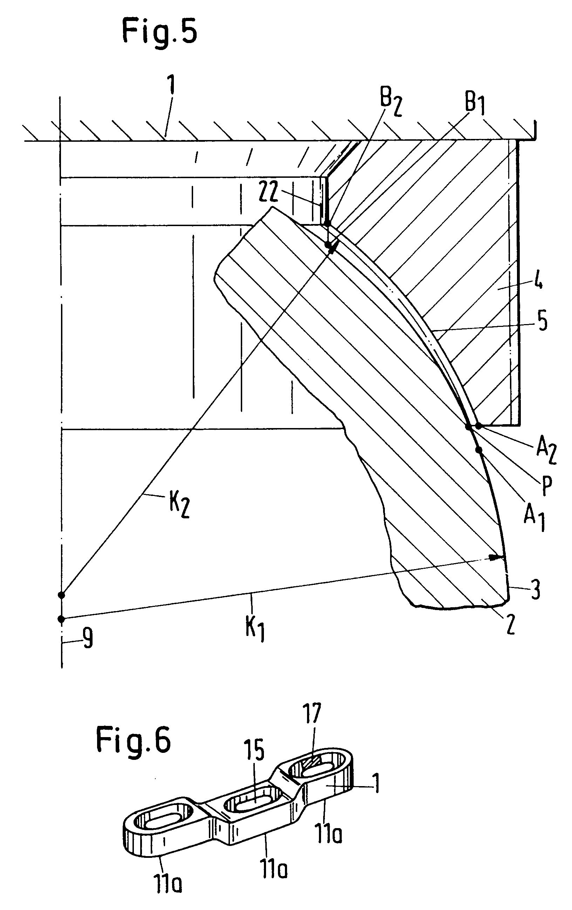

Figur 1 zeigt ein Verbindungssystem mit zwei Pedikelschrauben 2 die windschief zueinander stehen und die jeweils über Schrauben 6 und Gegenkörper 4 mit einem Verbindungsträger 1 verbunden sind. Der Verbindungsträger weist Langlöcher 15 auf, deren mittlerer Abstand in etwa entsprechend dem Abstand der Köpfe der Pedikelschrauben ausgewählt ist, zum Beispiel 25 mm. In Figur 2a besitzt der Verbindungsträger 1 im Bereich des Langloches 15 eine ebene Abstützfläche 11a an der die ebenfalls ebene Abstützfläche 11b des Gegenkörpers anliegt. Die Achse 9 der Pedikelschraube 2, in der eine Schraube 6 in den Kopf der Pedikelschraube einschraubbar ist, steht aus einer Senkrechten 12 zu den Abstützflächen 11a, 11b um einen Winkel 13 schräg gestellt. Der Kopf der Pedikelschraube 2 ist kugelförmig ausgebildet, wobei der Kugelkopf eine kugelförmige Abstützfläche 3 aufweist. Der Kugelkopf liegt auf einer Kalottenförmigen Abstützfläche 5 des Gegenkörpers 4 auf. Der Kopf der Schraube 6 hat ebenfalls eine Kugelabstützfläche deren Radius 18 auch dem Radius eines zylindrischen Flächenstücks 17 im Langloch 15 des Verbindungsträgers 1 entspricht. Bei der Schrägstellung aus der Senkrechten 12 können sich kugelförmige und kalottenförmige Abstutzflächen 3,5 sowie Kugelabstützfläche 7 und zylindrische Fläche 17 gegeneinander drehen während die Abstützflächen 11a, 11b sich gegeneinander in der Fläche verschieben. Sobald nun durch die Schraube 6 eine Vorspannung angelegt wird, würden sich die ebenen Abstützflächen 11a, 11b, wieder zurückbewegen, wenn ihre Verbindung nicht selbsthemmend wäre. In den Figuren 3 und 4 ist ein Verbindungsträger 1 mit zwei Langlöchern 15 gezeigt, die in einem mittleren Abstand 16 zueinander angeordnet sind. Die Langlöcher besitzen jeweils zwei Flächenstücke 17 aus einem Zylinder 19 dessen Achse 20 in der Längsrichtung des Verbindungsträgers verläuft, wobei der Radius 18 vom Zylinder 19 dem Radius der Kugelabstützfläche 7 am Schraubelement 6 entspricht, wie auch aus Figur 2 ersichtlich ist. Die Kalottenförmige Abstützfläche 5 im Gegenkörper 4 hat Untermass gegenüber der kugelförmigen Abstützfläche 3 der Pedikelschraube 2. In der stark verzerrten Darstellung der Figur 5 wird die kugelförmige Abstützfläche 3 mit Radius K1 in der Richtung der Achse 9 der Pedikelschraube 2 in den Gegenkörper eingefahren, bis sie an noch nicht aufgeweiteter Kontur 22 mit Radius K2 am Punkt P aufsitzt. In dieser Stellung sind Schwenkungen der Achse 9 noch möglich. Mit dem Anziehen von Schraubelement 6 wird der Gegenkörper 4 elastisch aufgeweitet und die Punkte A₁, B₁ der Kugelfläche verschieben sich zu den Punkten A₂, B₂ an der Kalottenfläche um die ganze Kalottenfläche 5 unter Vorspannung zum Tragen zu bringen. Die Kalottenfläche ist sandgestrahlt und besitzt eine aufgerauhte Oberfläche mit vorstehenden Spitzen welche unter Verformung in die Kugeloberfläche eindringen und somit grosse Biegemomente zwischen Pedikelschraube und Gegenkörper übertragbar machen.

Ähnlich ist die Situation der ebenen Abstützflächen 11a, 11b in den Figuren 2a und 3, die ebenfalls sandgestrahlt sind, um ein Gegeneinandergleiten in der Fläche unter Vorpannung zu verhindern. Dadurch dass sich die Mikrostruktur der Oberflächen gegenseitig durchdringt entsteht eine Selbsthemmung, die die Winkelabweichungen 13 der Pedikelschraube aus der Senkrechten 12 zulassen. Dieser Effekt kann verstärkt werden, indem die Abstützflächen 11a, 11b mit einer sich wiederholenden und sich gegenseitig durchdringenden Mikrostruktur versehen werden. Figur 7 zeigt Mikrostrukturen von Oberflächen 11a, wobei sich Rücksprünge in Form von Negativen zu Pyramiden- oder Kegelstümpfen in mindestens zwei verschiedenen Richtungen wiederholen. Wenn die Wiederholschritte entsprechend klein gewählt sind entsteht ein feiner Raster für die gegenseitige Verankerung der Abstützflächen 11a, 11b unter Vorspannung.

Die Anschrägung der Vorsprünge lässt im unbelasteten Zustand eine Verschiebung in der zu den Abstützflächen 11a und 11b übereinstimmenden Ebene zu. Vorsprünge in Pyramidenform, die Gegenstücke zur oberen Darstellung von Figur 7 sind, lassen sich als Teile und als Prägewerkzeuge für die Formumkehr besonders einfach herstellen.

Da die Kugelkalotte der Abstützfläche 5 und die kugelförmige Abstützfläche 3 ihr Drehzentrum praktisch beibehalten, kann der Durchbruch im Gegenkörper 4 so gewählt werden, dass das Schraubelement 6 mit der Pedikelschraube 2 nur in einem Winkel 13 verschwenkt werden kann, der der Selbsthemmung der Abstützflächen 11a, 11b unter Vorspannung entspricht.

In Figur 6 ist ein abgekröpfter Verbindungsträger 1 mit drei Langlöchern 15 und mit Ansenkungen gezeigt, die zylindrische Flächenstücke 17 für den Angriff der Schraubelemente aufweisen. Die Übergangsstücke zwischen zwei Langlöchern sind so ausgeführt, z. B. durch Reduktion des Querschnittes, dass ein plastisches Verbiegen zur Anpassung der Grobausrichtung der Abstützflächen 11a in einem Operationssaal mit Werkzeugen noch möglich ist. In einem solchen Fall ist es vorteilhaft wenn die Abstützflächen 11a aus ihrer Ebene nur Rücksprünge als Mikrostruktur aufweisen.

In der Figur 2b ist gegenüber der Figur 2a eine Schraube 6 mit längerem Gewindeteil 8 im Kopf der Pedikelschraube 2 verschraubt und ihrerseits durch einen Gegenkörper 4b mit kalottenförmiger Abstützfläche 5 zu ihrer Kugelabstützfläche 7 und mit ebener Abstützfläche 11c zum Verbindungsträger 1, der eine der Abstützfläche 11c gepaarte Abstützfläche 11a' aufweist, unterlegt. Auch hier kann die kalottenförmige Abstützfläche 5 Untermass aufweisen, um eine Vorspannung zu erzeugen. Auch hier können die Abstützflächen eine sich durchdringende Mikrostruktur aufweisen.

## Patentansprüche

1. Verbindungssystem für Pedikelschrauben, die in verschiedenen Wirbeln verankerbar sind, mit einem durchbrochenen Verbindungsträger (1), mit Pedikelschrauben (2), die einen Kopf mit kugelförmiger Abstützfläche (3) aufweisen, mit einem durchbrochenen Gegenkörper (4), der am Verbindungsträger aufliegt und auf der Gegenseite eine kalottenförmige Abstützfläche (5) mit Durchbruch (14) aufweist, wobei der Gegenkörper (4) mit seiner kalottenförmigen Abstützfläche (5) auf dem kugelförmigen Teil (3) am Kopf der Pedikelschraube aufliegt, und mit einem Schraubelement (6), welches eine Kugelabstützfläche (7) besitzt und über ein Gewinde (8) in Richtung einer Achse (9) der Pedikalschraube letztere mit dem Verbindungsträger (1) und dem Gegenkörper (4) verspannt,
dadurch gekennzeichnet, dass das Schraubelement (6) eine Schraube ist, die mit ihrem Schaft (10) im Kopf der Pedikelschraube verankerbar ist, dass der Gegenkörper auf der der kalottenförmigen Abstützfläche genenüberliegenden Seite auf einer ebenen mit dem Verbindungsträger (1) gemeinsamen Stützfläche beliebig verschiebbar ist, um die Pedikelschraube (2) mit ihrer Achse (9) in einem von der Senkrechten (12) auf die ebene Stützfläche abweichenden Winkel (13) über den Gegenkörper (4) am Verbindungsträger zu befestigen, wobei in der Schrägstellung aus der Senkrechten (12) zur ebenen Stützfläche Gegenkörper (4) und Verbindungsträger (1) selbsthemmend verbindbar sind.

2. Verbindungssystem nach Anspruch 1, dadurch gekennzeichnet, dass das Schraubelement (6) aus einem an der Pedikelschraube (2) angeformten Gewindestift und einer Gewindemutter mit Kugelabstützfläche (7) gebildet ist.

3. Verbindungssystem nach Anspruch 1 oder 2, dadurch gekennzeichnet, dass der Verbindungsträger Durchbrüche (14) in Form von Langlöchern (15) in einem Abstand (16) aufweist, der einem mittleren Abstand von zwei benachbarten Wirbeln beispielsweise 25 mm entspricht, und dass die Aufnahmefläche an den Langlöchern (15) für die Kugelabstützfläche (7) des Schraubelementes (6) aus zylindrischen Flächenstücken (17) besteht, die einem Zylinder (19) mit dem Radius (18) der Kugelabstützfläche (7) entsprechen, wobei der Zylinder (19) mit seiner Achse (20) in der Richtung der Aufweitung des Langlochs (15) verläuft.

4. Verbindungssystem nach Anspruch 1 oder 2 dadurch gekennzeichnet, dass der Verbindungsträger (1) Durchbrüche (14) in Form von Langlöchern (16) aufweist, die beidseitig mit ebenen Stützflächen (11a, 11a') versehen sind, und dass zwischen der Kugelabstützfläche (7) des Schraubelementes (6) und einer Stützfläche (11a') ein weiterer Gegenkörper (4b) verspannt ist, der eine analoge Funktion zum Gegenkörper (4) zwischen Pedikelschraube (2) und Verbindungsträger (1) ausübt.

5. Verbindungssystem nach einem der Ansprüche 1 bis 4, dadurch gekennzeichnet, dass die kalottenförmige Abstützfläche (5, 5b) am Gegenkörper (4, 4b) soviel Untermass aufweist, dass sie erst beim Anziehen der Verbindung durch das Aufweiten des Gegenkörpers (4, 4b) auf der Kugelabstützfläche (3, 7) zum Anliegen kommt, um über die unter Vorspannung ineinandergreifende Mikrostruktur der Oberflächen in jeder Richtung ein Drehen in der Kugelabstützfläche zu verhindern.

6. Verbindungssystem nach einem der Ansprüche 1 bis 5, dadurch gekennzeichnet, dass jeweils mindestens eine der miteinander gepaarten Abstützflächen (3, 5) (11a, 11b) (7, 17) (7, 5) (11a', 11c) aufgerauht oder mit einer Mikrostruktur versehen ist, um mit vorstehenden Spitzen und mit plastischer Deformation im Mikrobereich die Reibung zwischen den sich gegenseitig stützenden Flächen zu erhöhen.

7. Verbindungssystem nach einem der Ansprüche 1 bis 5, dadurch gekennzeichnet, dass die ebenen Abstützflächen (11a, 11b) (11a', 11c) eine sich durchdringende Mikrostruktur aufweisen die derart gerastert ist, dass der Gegenkörper (4, 4b) in der ebenen Stützfläche in zwei verschiedenen Richtungen gegenüber dem Verbindungsträger (1) in kleinen Schritten verschiebbar ist, wobei bei jedem Schritt ein Herausheben und ein Wiedereintauchen stattfindet, damit nach dem Eintauchen der sich durchdringenden Strukturen Schubkräfte in der ebenen Stützfläche übertragbar sind.

8. Verbindungssystem nach einem der Ansprüche 1 bis 7, dadurch gekennzeichnet, dass verschiedene Verbindungsträger (1) mit unterschiedlichem mittleren Abstand (16) der Langlöcher (15) vorgesehen sind.

9. Verbindungssystem nach einem der Ansprüche 1 bis 8, dadurch gekennzeichnet, dass verschiedene abgebogene Verbindungsträger (1) vorgesehen sind, deren ebene Abstützflächen (11a, 11a') von einem Langloch (15) zum anderen nicht parallel zueinander sind.

10. Verbindungssystem nach einem der Ansprüche 1 bis 9, dadurch gekennzeichnet, dass verschiedene Verbindungsträger (1) vorgesehen sind, deren Ebenen der Abstützflächen (11a, 11a') von einem Langloch (15) zum anderen zueinander versetzt sind.

11. Verbindungssystem nach einem der Ansprüche 1 bis 10, dadurch gekennzeichnet, dass Verbindungsträger (1) vorgesehen sind, deren Langlöcher (15) mit ihrer Längsachse in der Ebene der Abstützflächen (11a, 11a') zueinander versetzt sind.

12. Verbindungssystem nach einem der Ansprüche 1 bis 11, dadurch gekennzeichnet, dass Verbindungsträger (1) vorgesehen sind, die mehr als zwei Langlöcher (15) mit ebenen Abstützflächen (11a, 11a') in einem mittleren Abstand (16) aufweisen.

## Claims

1. Connection system for pedicle screws anchorable in different vertebrae, said connection system comprising an apertured load carrying link (1); pedicle screws (2) having a head with a spherical support surface (3); an apertured counter-body (4) which contacts the load carrying link and has on its opposite side a spherically concave support surface (5) with an aperture (14), with the counter-body (4) contacting the spherical part (3) at the head of the pedicle screw with its spherically concave support surface (5); and a screw element (6) which has a spherical support surface (7) and clamps the pedicle screw to the to the load carrying link (1) and the counter-body (4) via a thread (8) extending in the direction of an axis (9) of the pedicle screw,
**characterized in that** the screw element (6) is a screw having a shaft (10) which can be anchored in the head of the pedicle screw, in that the counter-body is displaceable as desired on the side opposite to the spherically concave support surface on a planar support surface common to the load carrying link (1) in order to secure the pedicle screw (2) to the load carrying link with the axis of the pedicle screw at an angle (13) which deviates from the perpendicular (12) to the planar support surface, wherein, in the position inclined relative to the perpendicular (12) to the planar support surface, the counter-body (4) and the load carrying link (1) are connectable in a self-locking manner.

2. Connection system in accordance with claim 1, characterized in that the screw element (6) is formed from a threaded pin formed on the pedicle screw (2) and a threaded nut with a spherical support surface (7).

3. Connection system in accordance with claim 1 or 2, characterized in that the load carrying link has apertures (14) in the form of elongate holes (15) which are separated by a distance (16) corresponding to an average separation between two neighbouring vertebrae, for example 25 mm, and in that the receiving surface at the elongate holes (15) for the spherical support surface (7 of the screw element (6) is comprised of cylindrical surface portions (17) corresponding to a cylinder (19) with a radius (18) equal to that of the spherical support surface (7), wherein the axis (20) of the cylinder (19) extends in the elongate direction of the elongate hole (15).

4. Connection system in accordance with claim 1 or 2, characterized in that the load carrying link (1) has apertures (14) in the form of elongate holes (16) which are provided with planar support surfaces (11a, 11a') on both sides, and in that a further counter-body (4b) is clamped between the spherical support surface (7) of the screw element (6) and a support surface (11a') and performs an analogous function to the counter-body (4) between the pedicle screw (2) and the load carrying link (1).

5. Connection system in accordance with one of the claims 1 to 4, characterized in that the spherically concave support surface (5, 5b) at the counter-body (4, 4b) is made sufficiently undersize that it first comes into contact with the spherical support surface (3, 7) after tightening the connection via a spreading deformation of the counter-body (4, 4b) in order to prevent a rotation in the spherical support surface in every direction via the microstructure of the surfaces which interengage under prestress.

6. Connection system in accordance with one of the claims 1 to 5, characterized in that in each case at least one of the mutually paired support surfaces (3, 5) (11a, 11b) (7, 17) (7, 5) (11a', 11c) is roughened or is provided with a microstructure in order to increase the friction between the mutually supporting surfaces via projecting points and via plastic deformation in the micro-region.

7. Connection system in accordance with one of the claims 1 to 5, characterized in that the planar support surfaces (11a, 11b) (11a', 11c) have an interpenetrating microstructure which is patterned in such a manner that the counter-body (4, 4b) is displaceable relative to the load carrying link (1) in small steps in the planar support surface in two different directions, wherein in each step a lifting out and reengagement takes place so that after the reengagement of the interpenetrating structures transverse forces can be transmitted in the planar support surface (11).

8. Connection system in accordance with one of the claims 1 to 7, characterized in that different load carrying links (1) with different average separations (16) of the elongate holes (15) are provided.

9. Connection system in accordance with one of the claims 1 to 8, characterized in that different bent load carrying links (1) are provided, the support surfaces (11a, 11a') of which are non-parallel to one another from one elongate hole (15) to the other.

10. Connection system in accordance with one of the claims 1 to 9, characterized in that different load carrying links (1) are provided, of which the planes of the support surfaces (11a, 11a') are offset relative to one another from one elongate hole (15) to the other.

11. Connection system in accordance with one of the claims 1 to 10, characterized in that load carrying links (1) are provided, of which the longitudinal axes of the elongate holes (15) are offset relative to one another in the plane of the support surfaces (11a, 11a').

12. Connection system in accordance with one of the claims 1 to 11, characterized in that load carrying links (1) are provided which have more than two elongate holes (15) with planar support surfaces (11a, 11a') at an average separation (16).

## Revendications

1. Système de connexion pour des vis pédiculaires qui peuvent être ancrées dans différentes vertèbres, avec un support de connexion percé (1), avec des vis pédiculaires (2) qui présentent une tête avec une surface d'appui sphérique (3), avec un contre-corps percé (4) qui repose sur le support de connexion et qui présente sur le côté opposé une surface d'appui (5) en forme de calotte avec un perçage (14), où le contre-corps (4) repose par sa surface d'appui (5) en forme de calotte sur la partie sphérique (3) à la tête de la vis pédiculaire, et avec un élément à visser (6) qui possède une surface d'appui sphérique (7) et qui serre par un filetage (8) en direction d'un axe (9) de la vis pédiculaire cette dernière avec le support de connexion (1) et le contre-corps (4), caractérisé en ce que l'élément à visser (6) est une vis qui peut être ancrée par son fût (10) dans la tête de la vis pédiculaire, en ce que le contre-corps, au côté opposé à la surface d'appui en forme de calotte peut être déplacé sélectivement sur une surface d'appui plane commune avec le support de connexion (1) pour fixer la vis pédiculaire (2) avec son axe (9) suivant un angle (13) s'écartant de la verticale (12) à la surface d'appui plane par le contre-corps (4) au support de connexion, où dans la position oblique à partir de la verticale (12) vers la surface d'appui plane, le contre-corps (4) et le support de connexion (1) peuvent être reliés d'une manière autobloquante.

2. Système de connexion selon la revendication 1, caractérisé en ce que l'élément à visser (6) est constitué d'une tige filetée rapportée par formage à la vis pédiculaire (2) et d'un écrou de filetage avec une surface d'appui sphérique (7).

3. Système de connexion selon la revendication 1 ou 2, caractérisé en ce que le support de liaison présente des perçages (14) sous la forme de trous oblongs (15) suivant un écart (16) qui correspond à un écart moyen de deux vertèbres avoisinantes par exemple de 25mm et en ce que la surface de réception aux trous oblongs (15) pour la surface d'appui sphérique (7) de l'élément à visser (6) est constituée de pièces de surface cylindriques (17) qui correspondent à un cylindre (19) ayant le rayon (18) de la surface d'appui sphérique (7), où le cylindre (19) s'étend avec son axe (20) dans la direction de l'élargissement du trou oblong (15).

4. Système de connexion selon la revendication 1 ou 2, caractérisé en ce que le support de connexion (1) présente des perçages (14) sous la forme de trous oblongs (16) qui sont pourvus des deux côtés de surfaces d'appui planes (11a, 11a') et en ce qu'il est serré entre la surface d'appui sphérique (7) de l'élément à visser (6) et une surface d'appui (11a') un contre-corps supplémentaire (4b) qui exerce une fonction analogue au contre-corps (4) entre la vis pédiculaire (2) et le support de connexion (1).

5. Système de connexion selon l'une des revendications 1 à 4, caractérisé en ce que la surface d'appui (5, 5b) en forme de calotte présente au contre-corps (4, 4b) une dimension inférieure de telle sorte que seulement lors du serrage de la connexion par l'élargissement du contre-corps (4, 4b) elle vient à s'appliquer à la surface d'appui sphérique (3, 7) pour empêcher par la microstructure, s'interpénétrant sous précontrainte, des surfaces dans toute direction une rotation dans la surface d'appui sphérique.

6. Système de liaison selon l'une des revendications 1 à 5, caractérisé en ce que, respectivement au moins l'une des surfaces d'appui appariées l'une à l'autre (3, 5) (11a, 11b) (7, 17) (7, 5) (11a', 11c) est rendue rugueuse ou est pourvue d'une microstructure pour augmenter par des pointes en saillie et par une déformation plastique dans la micro-zone le frottement entre les surfaces se supportant mutuellement.

7. Système de connexion selon l'une des revendications 1 à 5, caractérisé en ce que les surfaces d'appui planes (11a, 11b) (11a', 11c) présentent une microstructure d'interpénétration qui est réalisée de façon que le contre-corps (4, 4b) dans la surface d'appui plane soit déplaçable dans deux directions différentes par rapport au support de connexion (1) par de petits pas, et à chaque pas ayant lieu une sortie et une remise en place pour que après l'engagement des structures qui s'interpénètrent, des forces de poussée puissent être transmises dans la surface d'appui plane.

8. Système de connexion selon l'une des revendications 1 à 7, caractérisé en ce que sont prévus différents supports de connexion (1) avec des écarts moyens différents (16) des trous oblongs (15).

9. Système de connexion selon l'une des revendications 1 à 8, caractérisé en ce que sont prévus différents supports de connexion pliés (1) dont les surfaces d'appui planes (11a, 11a') ne sont pas parallèles l'une à l'autre d'un trou oblong (15) vers l'autre.

10. Système de connexion selon l'une des revendications 1 à 9, caractérisé en ce que sont prévus différents supports de connexion (1) dont les plans des surfaces d'appui (11a, 11a') sont décalés d'un trou oblong (15) vers l'autre.

11. Système de connexion selon l'une des revendications 1 à 10, caractérisé en ce que des supports de connexion (1) sont prévus dont les trous oblongs (15) avec leur axe longitudinal sont décalés les uns aux autres dans le plan des surfaces d'appui (11a, 11a').

12. Système de connexion selon l'une des revendications 1 à 11, caractérisé en ce que sont prévus des supports de connexion (1) qui présentent plus de deux trous oblongs (15) avec des surfaces d'appui planes (11a, 11a') suivant un écart moyen (16).
